# EUROPEAN PATENT APPLICATION

(11) **EP 2 014 322 A1**
(43) Date of publication of application: **14.01.2009**
(21) Application number: 06790974.7
(22) Date of filing: 12.09.2006
(51) Int. Cl.: A61M 5/178

(54) **A STERILE DRUG-MIXING SYRINGE**

(30) Priority: 18.04.2006 CN 200610042690; 18.04.2006 CN 200620078828 U; 25.05.2006 CN 200620079041 U
(71) Applicant: ZHONGSHAN BOTAI PHARMACEUTIC INSTRUMENTS CO., LTD., Zhongshan Guangdong 528437 (CN)
(72) Inventor: HUO, Pingan, Guangdong 528437 (CN); ZHANG, Wenqing, Guangdong 528437 (CN); LI, Wuyuan, Guangdong 528437 (CN); LIU, Yan, Guangdong 528437 (CN); WANG, Xinming, Guangdong 528437 (CN); JIN, Chunqing, Guangdong 528437 (CN)
(74) Representative: Needle, Jacqueline
(86) International application number: PCT/CN2006/002361
(87) International publication number: WO 2007/118372

(57) **Abstract**

A menstruum prepositioned type sterile drug-mixing syringe and a menstruum separately positioned type sterile drug-mixing syringe include a shell (9), a needle (5), a piston (10) and a push-pull rod assembly, further include a menstruum bottle (3), a solute bottle (8), a sliding sleeve (2) and a needle loading means. The menstruum bottle (3) is fixed to the inner of a sliding sheath (2), the solute bottle (8) is fixed to the inner of the shell (9), the piston (10) is disposed in the mouth of the bottom of the solute bottle (8), the sliding sleeve (2) is socketed to the front end of the shell (9) in advance or disposed by other ways, the mouth of the menstruum bottle (3) is opposite to the mouth of the solute bottle (8), the middle thereof is connected by the needle loading means, the needle (5) is a communicating needle which has piercing tips on the two ends, the needle punctures the needle loading means and securely connects to it.

## Description

### TECHNICAL FIELD

The present invention relates to a syringe for medical use, more particularly, to a menstruum prepositioned type sterile drug-mixing syringe which combines the menstruum, the solute and the syringe as a whole and a menstruum separately positioned type sterile drug-mixing syringe.

### BACKGROUND ART

In recent years, the applicant of the present invention has made significant improvements to the constructions of conventional syringes in view of the deficiencies found in the injection operation and has filed several patent applications for pre-charged type syringes which can reduce workload due to their simplified injection operation, and more importantly, avoid occurrence of possible medical accidents caused by mixing the wrong drugs. Said pre-charged type syringes also have the advantage of automatic injection and are convenient to use. However, in most said pre-charged type syringes the menstruum vial disadvantageously contacts the push-pull of the syringe, and the solute cartridge (must be of the cartridge type) is positioned on the front side of the syringe. The drug mixing operation is achieved when the menstruum under an inner pressure is forced into the solute cartridge through a communicating needle, then the mixed drug solution is pushed out of the solute cartridge. Although said pre-charged type syringes have certain advantages over conventional syringes, further improvements are necessary. For example, in addition to the injection needle, a communication needle is required. This will complicate the manufacturing process and increase cost.

Chinese patent application No. 200610042690.2 filed by the applicant on April 18, 2006 entitled "Menstruum Prepositioned Type Sterile Drug Mixing Syringe for Cartridges" discloses a pre-charged type syringe having a prepositioned menstruum vial in which the solute vial contacts the push-pull and the menstruum vial (and its sliding sleeve) is positioned in front of the syringe. Injection can be performed simply by removing the menstruum vial (and its sliding sleeve) after the drug is mixed and the communicating needle is omitted. This will simplify both the drug mixing procedure and the structure of the syringe.

Chinese patent application No. 200620078828.X filed by the applicant on April 18, 2006 entitled "Liquid Drug Syringe for Cartridges" discloses a pre-charged type syringe for liquid drugs having a two-stage push-pull, significantly reducing the total length of the push-pull thus minimizing the packing size so that the syringe is convenient to carry and transport.

Chinese patent application No. 200620079041.5 filed by the applicant on May 25, 2006 entitled "Menstruum Separately Positioned Type Sterile Drug Mixing Syringe for Cartridges" discloses a pre-charged type syringe having a menstruum vial (and its sliding sleeve) separated from the syringe characterized in that the menstruum (and its sliding sleeve) is connected to the front of the syringe only when the drug mixing operation starts, further reducing the overall length of the syringe. Thus the packing size of the syringe is also reduced, even more convenient to carry and transport.

The present invention claims priorities of the above-mentioned 3 Chinese patent applications.

### SUMMAY OF THE INVENTION

It is an object of the present invention to overcome the disadvantages of the existing pre-charged type syringes by providing a menstruum prepositioned type sterile drug-mixing syringe which combines the menstruum, the solute and the syringe as a whole in order to make the injection operation simple, convenient and fast.

It is another object of the present invention to provide a menstruum separately positioned type sterile drug-mixing syringe so that not only the injection operation is simplified and made more convenient, but also the packing size is reduced so that the syringe is more convenient to carry.

The objects of the menstruum prepositioned type sterile drug-mixing syringe according to the present invention are achieved by the following technical solutions:
The menstruum prepositioned type sterile drug-mixing syringe according to the present invention comprises: a shell of the syringe, a needle positioned in the front end of the shell, a piston inside the opening at the rear end of the shell and a push-pull assembly connected to the piston, in which:
   the menstruum prepositioned type sterile drug-mixing syringe further comprises a sliding sleeve, a menstruum bottle, a solute bottle and a needle loading means; in which:
      the sliding sleeve is a hollow cylinder and the menstruum bottle is fixedly socketed in the front portion inside the sliding sleeve;
      the shell is a hollow cylinder and the front portion of the shell is slidably socketed in the rear portion of the sliding sleeve;
      the solute bottle is a cartridge pre-charged with powder drug and the piston is slidably socketed in the opening bottom of the solute bottle, which is fixedly socketed in the shell;
      the mouth of the menstruum bottle facing the mouth of the solute bottle and the tow bottles is connected through the needle loading means;
      the needle is a hollow needle having piercing tips at both ends, piercing into the needle loading means and fixedly coupled to the needle loading means.

The objects of the menstruum prepositioned type sterile drug-mixing syringe according to the present invention are achieved by the following technical solutions:
The menstruum prepositioned type sterile drug-mixing syringe according to the present invention, in which the needle loading means comprising a sliding support, a middle support and a needle support, in which:
   the sliding support comprising two sections of hollow cylinders, the diameter of the front section is greater than the diameter of the rear section and a circular step is formed at the joint of the two sections of hollow cylinders having different diameters; the mouth of the menstruum bottle is tightly engaged with the inner wall of the front portion of the sliding support and is restricted to inner side of the circular step, and; the rear portion of the sliding support is slidably socketed with the middle support;
   the middle support is a hollow cylinder; the front portion of the middle support slidably socketed with the sliding support and the rear end of the middle support coaxially contacting the front end of the needle support;
   the sliding support and the middle support is provided with a pair of positioning mechanism engaging with each other;
   the needle support is a hollow cylinder; the front end of the needle support is closed having a bore formed in the centre, through which the needle passes and the fixedly connects to the needle support; an non-return mechanism is formed on the inner wall at the rear portion of the needle support and the rear portion of the needle support slidably socketing with the mouth of the solute bottle.

The menstruum prepositioned type sterile drug-mixing syringe according to the present invention, in which said a pair of positioning mechanism is a circular bulge or groove formed on the surface of the sliding support slidably contacting the middle support and a circular groove or bulge formed on the surface of the middle support slidably contacting the sliding support.

The menstruum prepositioned type sterile drug-mixing syringe according to the present invention, in which said a pair of positioning mechanism is a protrusion or an indent formed on the surface of the sliding support slidably contacting the middle support and an indent or a protrusion formed on the surface of the middle support slidably contacting the sliding support.

The menstruum prepositioned type sterile drug-mixing syringe according to the present invention, in which the non-return mechanism is claws or a collar formed on the inner wall in the rear portion of the needle support.

The menstruum prepositioned type sterile drug-mixing syringe according to the present invention, in which the push-pull means comprising a first stage push-pull, a second stage push-pull, a handle and a fixing sleeve, in which;
the first stage push-pull is a hollow cylinder; a circular step bulging outward is formed on the front end of the first stage push-pull; the fixing sleeve having a reducing rear end, the front end of the fixing sleeve is put on the circular step and screwed to or tightly coupled to the rear end of the shell in order to coaxially fix the first stage push-pull to the rear end of the shell;
the second stage push-pull is slidably socketed in the first stage push-pull, claws is provided on the front end of the second stage push-pull; the rear end of the second stage push-pull is fixedly connected to the handle.

The menstruum prepositioned type sterile drug-mixing syringe according to the present invention, in which an indenter having an outer diameter slightly smaller than the inner diameter of the solute bottle is tightly coupled to the front end of the first stage push-pull. The central protruding end of the indenter is embedded into the rear end of the piston in the solute bottle.

The menstruum prepositioned type sterile drug-mixing syringe according to the present invention, in which a positioning ring having an outer diameter equal to or slightly smaller than the inner diameter of the shell is covered on the indenter.

The menstruum prepositioned type sterile drug-mixing syringe according to the present invention, in which the menstruum is a vial pre-charged with liquid menstruum and compressed air, or a cartridge pre-charged with liquid menstruum without inner pressure.

The menstruum prepositioned type sterile drug-mixing syringe according to the present invention, in which the front end of the sliding sleeve is provided with an end cap, a protrusion or a circular bulge formed in the middle portion inside the sliding sleeve to hold the shoulder of the menstruum bottle.

The objects of the menstruum separately positioned type sterile drug-mixing syringe according to the present invention are achieved by the following technical solutions:
The menstruum separately positioned type sterile drug-mixing syringe according to the present invention comprises a shell of the syringe, a needle positioned in the front end of the shell, a piston inside the opening at the rear end of the shell and a push-pull assembly connected to the piston, in which:
   the menstruum separately positioned type sterile drug-mixing syringe further comprising a menstruum bottle and a solute bottle and a needle loading means;
   the shell is a hollow cylinder;
   the solute bottle is a cartridge pre-charged with powder drug, the piston is slidably socketed in the opening bottom of the solute bottle, the solute bottle is fixedly socketed in the shell, the mouth of the solute bottle facing forwardly;
   the menstruum bottle is separately positioned with other parts;
   the rear end of the needle loading means is slidably socketed on the mouth of the solute bottle;
   the needle is a hollow needle having piercing tips at both ends, piercing into the needle loading means and fixedly coupled to the needle loading means.

The objects of the menstruum separately positioned type sterile drug-mixing syringe according to the present invention are further achieved by the following technical solutions:
The menstruum separately positioned type sterile drug-mixing syringe according to the present invention further comprises a sliding sleeve, the inner wall at the rear portion of the sliding sleeve slidably engaging with the outer wall at the front portion of the shell, the menstruum bottle is fixedly socketed in the front portion of the sliding sleeve, the mouth of the menstruum bottle facing backwardly.
The menstruum separately positioned type sterile drug-mixing syringe according to the present invention, in which the front end of the sliding sleeve is provided with a end cap, a protrusion or a circular bulge is form in the middle portion inside the sliding sleeve to hold the shoulder of the menstruum bottle.
The menstruum separately positioned type sterile drug-mixing syringe, further comprises a packing body, the sliding sleeve and the menstruum bottle fixed in the sliding sleeve is put in a recess of the packing body, and other components of the syringe is put in a recess of the packing body.
The menstruum separately positioned type sterile drug-mixing syringe according to the present invention, in which the needle loading means comprising a needle support, in which the needle support is a hollow cylinder, the front end of the needle support is closed having a bore formed in the centre, the needle passing through the bore and fixedly connected to the needle support, non-return mechanism is formed on the inner wall at the rear portion of the needle support, the rear portion of the needle support slidably socketing with the mouth of the solute bottle.
The menstruum separately positioned type sterile drug-mixing syringe according to the present invention, in which the non-return mechanism is claws or a collar formed on the inner wall in the rear portion of the needle support.
The menstruum separately positioned type sterile drug-mixing syringe according to the present invention, in which the push-pull means comprising a first stage push-pull, a second stage push-pull, a handle and a fixing sleeve, in which;
   the first stage push-pull is a hollow cylinder; a circular step bulging outward is formed on the front end of the first stage push-pull, the fixing sleeve having a reducing rear end; the front end of the fixing sleeve is put on the circular step and screwed to or tightly coupled to the rear end of the shell in order to coaxially fix the first stage push-pull to the rear end of the shell;
   the second stage push-pull is slidably socketed in the first stage push-pull; claws is provided on the front end of the second stage push-pull; the rear end of the second stage push-pull is fixedly connected to the handle.
The menstruum separately positioned type sterile drug-mixing syringe according to the present invention, in which an indenter having an outer diameter slightly smaller than the inner diameter of the solute bottle is tightly coupled to the front end of the first stage push-pull. The central protruding end of the indenter is embedded into the rear end of the piston 10 in the solute bottle.
The menstruum separately positioned type sterile drug-mixing syringe according to the present invention, in which a positioning ring having an outer diameter equal to or slightly smaller than the inner diameter of the shell is covered on the indenter.
The menstruum separately positioned type sterile drug-mixing syringe according to the present invention, in which the menstruum is a vial pre-charged with liquid menstruum and compressed air, or a cartridge pre-charged with liquid menstruum without inner pressure.
The menstruum separately positioned type sterile drug-mixing syringe according to the present invention, in which the needle is covered with a protection jacket.

### BRIEF DESCRIPTION OF THE DRAWINGS

FIG. 1 is a cross-section view showing the initial stage of the menstruum prepositioned type sterile drug-mixing syringe;
FIG. 2 is a cross-section view of the menstruum prepositioned type sterile drug-mixing syringe showing the needle piercing into the rubber stopper of the solute bottle;
FIG. 3 is a cross-section view of the menstruum prepositioned type sterile drug-mixing syringe showing the two ends of the needle piercing into the rubber stoppers of the solute bottle and the menstruum bottle respectively for drug mixing;
FIG. 4 is a cross-section view of the menstruum prepositioned type sterile drug-mixing syringe showing the sliding support and the middle support of the menstruum bottle and the needle loading means being removed and the second stage push-pull being pulled out from the first stage pushing stage after drug mixing being completed; FIG. 4 is also a cross-section view of a menstruum separately positioned type sterile drug-mixing syringe showing the menstruum bottle being removed and the second stage push-pull being pulled out from the first stage pushing stage after drug mixing being completed;
FIG. 5 is a cross-section view of the menstruum prepositioned type sterile drug-mixing syringe and the menstruum separately positioned type sterile drug-mixing syringe showing the injection operation being completed;
FIG. 6 is a cross-section view showing components of the needle loading means of the menstruum prepositioned type sterile drug-mixing syringe;
FIG. 7 is a cross-section view showing the initial stage of the needle loading means of the menstruum prepositioned type sterile drug-mixing syringe;
FIG. 8 is a cross-section view showing the drug mixing stage of the needle loading means of the menstruum prepositioned type sterile drug-mixing syringe;
FIG. 9 is a cross-section view showing components of the push-pull assembly of the menstruum prepositioned type sterile drug-mixing syringe and the menstruum separately positioned type sterile drug-mixing syringe;
FIG. 10 is a cross-section view showing the initial stage of the push-pull assembly of the menstruum prepositioned type sterile drug-mixing syringe and the menstruum separately positioned type sterile drug-mixing syringe;
FIG. 11 is a cross-section view showing the push-pull assembly of the menstruum prepositioned type sterile drug-mixing syringe and the menstruum separately positioned type sterile drug-mixing syringe when the second stage push-pull being pulled out form the first stage push-pull;
FIG. 12 is a perspective view of the menstruum separately positioned type sterile drug-mixing syringe showing the menstruum bottle in the sliding sleeve;
FIG. 13 is a cross-section view showing the initial stage of the menstruum separately positioned type sterile drug-mixing syringe without the menstruum bottle and the sliding sleeve;
FIG. 14 is a perspective view of the menstruum separately positioned type sterile drug-mixing syringe showing the sliding sleeve containing the menstruum bottle being put on the front part of the syringe ready for drug mixing;
FIG. 15 is a cross-section view of the menstruum separately positioned type sterile drug-mixing syringe showing the two ends of the needle piercing into the rubber stoppers of the solute bottle and the menstruum bottle respectively for drug mixing;
FIG. 16 is a cross-section view showing the packed menstruum separately positioned type sterile drug-mixing syringe.

### DESCRIPTION OF THE PREFERRED EMBODIMENT

Hereinafter, the menstruum prepositioned type sterile drug-mixing syringe and the menstruum separately positioned type sterile drug-mixing syringe according to the present invention will be described with reference to the accompanying drawings.

### 1. Menstruum prepositioned type sterile drug-mixing syringe

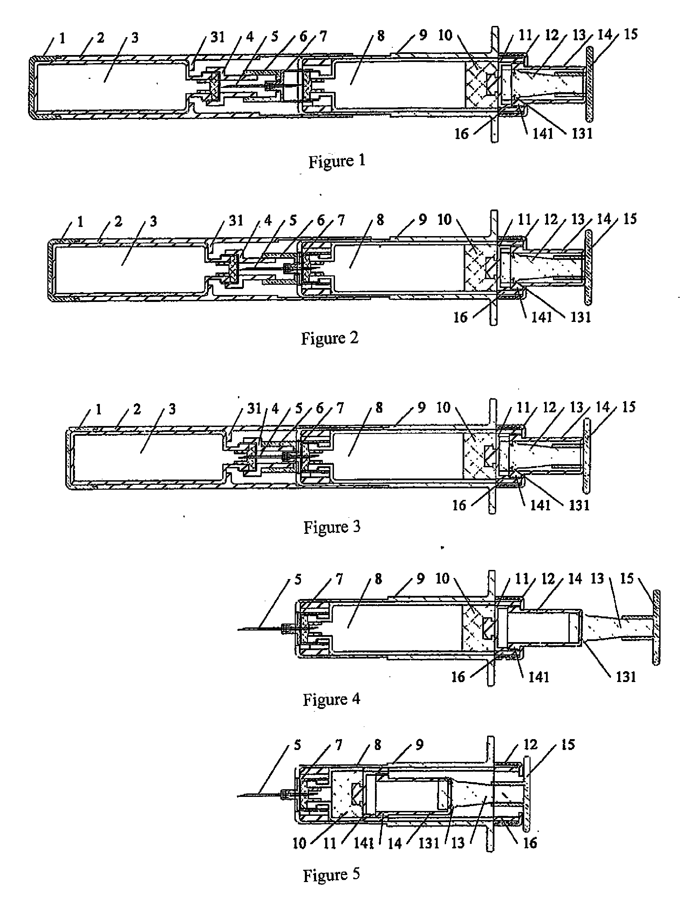

Referring to FIG. 1 through FIG. 11, the menstruum prepositioned type sterile drug-mixing syringe of the present invention comprises a sliding sleeve 2, a menstruum bottle 3, a solute bottle 8, a piston 10, a shell 9, a needle 5, a needle loading means and a push-pull assembly, in which:
The sliding sleeve 2 is a hollow cylinder provided with an end cap 1 in its front end and a circular bulge 31 in the middle inside the sliding sleeve 2. The menstruum 3 is a vial sealed inside the sliding sleeve 2 via the end cap 1 screwed to or tightly coupled to the sliding sleeve 2. The mouth of the menstruum bottle 3 faces backwardly and the bottle shoulder rests against the circular bulge 31 formed inside the sliding sleeve 2;
The solute bottle 8 is a cartridge and the piston 10 is positioned inside the solute bottle 8 from the opening bottom of the solute bottle 8;
The shell 9 is a hollow cylinder having a circular step formed inside the shell 9 in its front portion. The solute bottle 8 is positioned inside the shell 9 with its mouth facing forward and its shoulder resting against the circular bulge formed inside the shell 9 in the front. The front portion of the shell 9 is slidably socketed into the rear opening of the sliding sleeve 2 so that the mouth of the menstruum bottle 3 faces to the mouth of the solute bottle 8 and the two mouths are communicated through the needle loading means;
The needle 5 is a communicating needle having piercing tips at both ends and pierces into the needle loading means and fixed thereto.

The needle loading means comprises a sliding support 4, a middle support 6 and a needle support 7, in which:
The sliding support 4 is composed of two sections of hollow cylinders of different diameter. The front section has a larger diameter than the rear section and a step is formed at the joint of the two sections. A circular bulge 41 is formed on the outer surface of the sliding support 4 at its rear end. The mouth of the menstruum bottle 3 tightly engages into the front inner wall of the sliding support 4 and is restricted to the inner side of its circular step. The outer surface of the rear portion of the sliding support 4 is slidably socketed with the inner surface of the front portion of the middle support 6;
The middle support 6 is a hollow cylinder having a circular groove 61 to engage with the circular bulge 41 formed on the outer surface of the rear portion of the sliding support 4. The rear end of the middle support 6 coaxially contacts the closed end of the needle support 7;
The needle support 7 is a hollow cylinder having a closed front end. A bore is formed in the centre of said closed front end through which the needle 5 passes and fixed to the needle support 7. The rear end of the needle support 7 is slidably socketed with the mouth of the solute bottle 8. Claws 71 are formed on the inner wall of the rear end of the needle support 7. Claws 71 engages to the backside of the mouth of the solute bottle 8 after it reaches to the closed end of the needle support 7 from the rear end of the needle support 7.

The push-pull assembly comprises a first stage push-pull 14, a second stage push-pull 13, a handle 15 and a fixing sleeve 12, in which:
The first stage push-pull 14 is a hollow cylinder having a protruding step 141 formed on the outer surface at its front end. The fixing sleeve 12 is put on the step 141 and screwed to the rear end of the shell 9 so as to coaxially fix the first stage push-pull to the rear end of the shell 9. An indenter having an outer diameter slightly smaller than the inner diameter of the solute bottle 8 is tightly coupled to the front end of the first stage push-pull 14. The central protruding end of the indenter 11 is embedded into the rear end of the piston 10 in the solute bottle 8. A positioning ring 16 having an outer diameter equal to or slightly smaller than the inner diameter of the shell 9 is covered on the indenter 11;

The cross section of the second stage push-pull 13 has a shape similar to a "+" or "Y" symbol (not shown). Tubular shape is also acceptable. The push-pull 13 is slidably socketed inside the first stage push-pull 14. Claws 131 are formed on the outside near the front portion of the second stage push-pull 13 and the rear end of the second stage push-pull 13 is fixedly coupled to the handle 15.

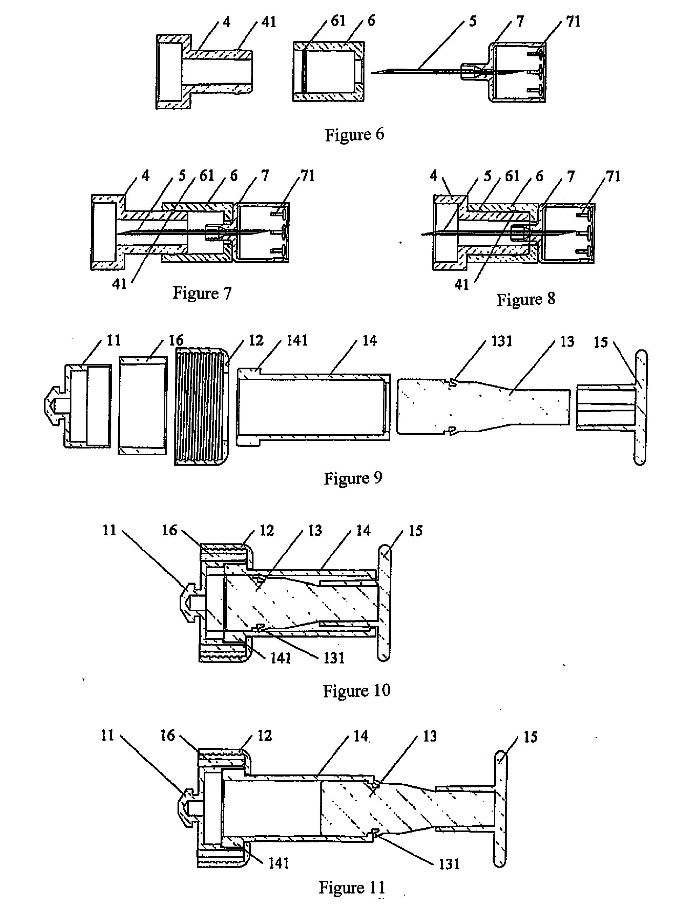

Before leaving factory, the menstruum bottle 3 of menstruum prepositioned type sterile drug-mixing syringe of the present invention is pre-charged and sealed with pre-determined amount of menstruum (e.g. water for injection, etc.) and pressurized with compressed air, and the solute bottle 8 is pre-charged and sealed with powder or liquid drug of predetermined dosage. At this time the whole syringe system is in an initial status as shown in FIG. 1. The sliding support 4, the middle support 6 and the needle support 7 of the needle loading means are in their initial positions as shown in FIG. 7. The circular bulge 41 on the outer surface of the sliding support 4 engages with the circular groove 61 in the inner wall of the middle support 6. The mouth of the solute 8 is in the rear portion of the needle support 7 but not completely passing through the claws 71 formed on the inner wall of the needle support 7. At this time the needle 5 in the middle position is not communicating the menstruum bottle 3 and the solute bottle 8. The push-pull assembly is now in its initial status as shown in FIG. 10. The overall length of the push-pull assembly is minimum as the second stage push-pull 13 is completely socketed in the first stage push-pull 14. At this time the claws 131 are inactive.

Before performing the injection operation using the menstruum prepositioned type sterile drug-mixing syringe of the present invention, point the front end of the syringe upwardly to make the syringe upright so that the menstruum bottle 3 in the sliding sleeve 2 stands downwardly. Then push the sliding sleeve 2 downwardly so that the menstruum bottle 2 will consequently push the sliding support 6 of the needle loading means to move. Because the circular bulge 41 of the sliding support has engaged with the circular groove 61 of the middle support 6, the middle support 6 is forced to move. The middle support 6 consequently forces the needle support 7 to move downwardly, so that the lower end of the needle 5 fixed to the needle support 7 firstly pierces through the rubber stopper of the solute bottle 8 (see FIG. 2). At this time the inner end of the needle support 7 biases against the mouth of the solute bottle 8 and the claws 71 inside the needle support 7 engages with the backside of the mouth of the solute bottle 8. Continue to push the sliding sleeve 2 downwardly so that it slides along the outer wall of the shell 9 until the sliding sleeve 2 reaches to its dead point. At this time the circular bulge 41 of the sliding support disengages with the circular groove 61 of the middle support 6 and the outside of the step of the sliding support 4 biases against the front end of the middle support 6 (see FIG. 8), now the upper end of the needle 5 pierces through the rubber stopper of the menstruum bottle 3 so that the menstruum bottle 3 communicates with the solute bottle 8 (see FIG. 3). Then the liquid menstruum will enter into the solute bottle 8 through the hollow needle 5 under the pressure inside the menstruum bottle 3 to mix with and dissolve the powder drug contained in the solute bottle 8.

When the drug mixing operation is completed, remove the sliding sleeve 2 so that the sliding support 4, the middle support 6 will disengage with the shell 9. The needle support 7 will not disengage with the shell 9 because the claws 71 of the needle support 7 now engages with the backside of the mouth of the solute bottle 8. Also the lower end of the needle 5 will not be removed from the rubber stopper of the solute bottle 8. At this time the upper end of the needle 5 is fully exposed. Now pull the handle 15 downwardly so that the second stage push-pull 13 will slide downwardly with respect to the first stage push-pull 14 until the claws 131 of the second stage push-pull 13 engages to the rear end of the first stage push-pull 14 (see FIG. 4). Then push the handle 15 to move the piston 10 via the second stage push-pull 13, the first stage push-pull 14 and the indenter 11 to remove the air inside the solute bottle 8. Now the syringe is ready for injection. The mixed and dissolved drug solution is injected into a human body through the needle 5 to complete the whole operation.

The menstruum bottle 3 is not necessary to be pressurized. In case of the menstruum bottle 3 without inner pressure, pull out the second stage push-pull 13 in advance and push the handle 15 to make the indenter 11 move the piston 10 upwardly. When the two bottles are communicated as shown in FIG. 3, pull the handle 15 downwardly to move the piston 10 downwardly so that the menstruum contained in the menstruum bottle 3 is drawn into the solute bottle 8 for drug mixing.

### 2. Menstruum separately positioned type sterile drug-mixing syringe

The menstruum separately positioned type sterile drug-mixing syringe of the present invention are similar to the menstruum prepositioned type sterile drug-mixing syringe as described above. Only the differences will be discussed.

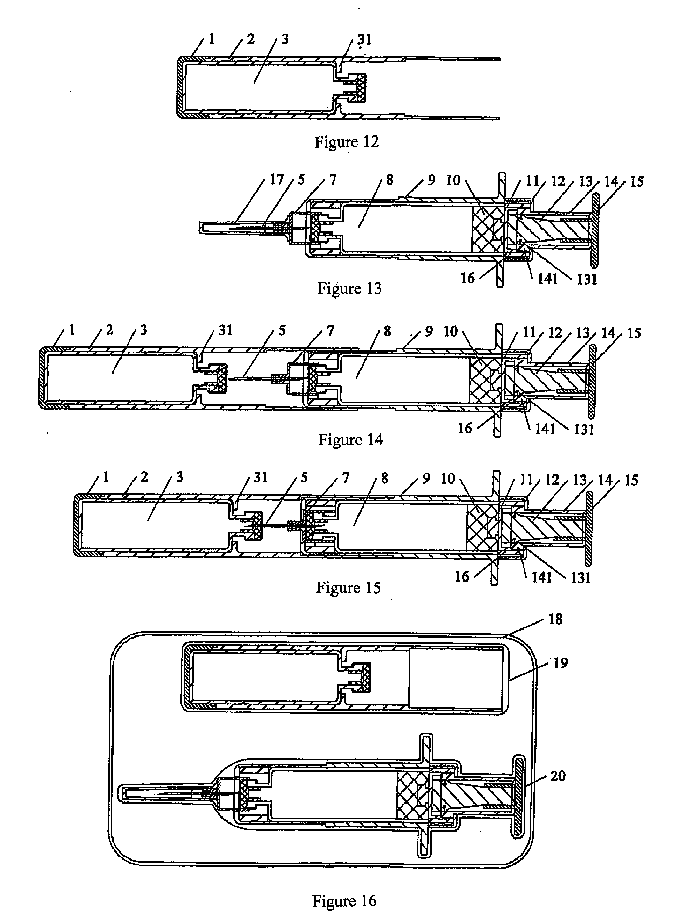

Referring to FIG. 12 - FIG. 16 and FIG. 4, 5, the menstruum separately positioned type sterile drug-mixing syringe of the present invention also comprises a sliding sleeve 2, a menstruum bottle 3, a solute bottle 8, a piston 10, a shell 9, a needle 5, a needle loading means and a push-pull assembly. The differences are that:
The sliding sleeve 2 containing the menstruum bottle 3 is not socketed with the shell 9(see FIG. 12), but is put in the recess 19 in the packing 18. Other components of the syringe are put in the recess 20 in the packing 18 (see FIG. 16);
The needle loading means comprises only a needle support 7. The sliding support 4 and the middle support 6 are omitted. The exposed end of the needle 5 is covered with a needle jacket 17 (see FIG. 13).

Other portions of the menstruum separately positioned type sterile drug-mixing syringe are identical to the menstruum prepositioned type sterile drug-mixing syringe in their shapes, structures, sizes and configurations.

Before injection, take out the sliding sleeve 2 containing the menstruum bottle 3 and other parts of the syringe from the packing. Then remove the needle jacket 17 from the needle 5. Socket the rear opening end of the sliding sleeve 2 into the front portion of the shell 9, then make the mouth of the menstruum bottle 3 facing downwardly and push the sliding sleeve 2 until the two ends of the needle 5 pierces into the rubber stoppers of the menstruum bottle 3 and the solute bottle 8 respectively. The following up procedures are identical to that of the menstruum prepositioned type syringe and will not be repeated herewith.

The sliding sleeve 2 can also be omitted and the mouth of the menstruum bottle can be directly pointed to the front end of the needle 5 and pushed to complete the drug mixing operation. Then the menstruum bottle 3 can be removed for the air removing and the injection operation.

Similarly, the menstruum bottle 3 is not necessary to be pressurized. In case of the menstruum bottle 3 without inner pressure, pull out the second stage push-pull 13 in advance and push the handle 15 to make the indenter 11 move the piston 10 upwardly. When the two bottles are communicated, pull the handle 15 downwardly to move the piston 10 downwardly so that the menstruum contained in the menstruum bottle 3 is drawn into the solute bottle 8 for drug mixing. Then remove the menstruum bottle 3 for the following up air discharging and injection operation.

Exemplary embodiments of the present invention have been disclosed herein and, it will be understood by those of ordinary skill in the art that various changes in form and details may be made without departing from the spirit and scope of the present invention as set forth in the following claims.

### Industrial Applicability

The menstruum prepositioned type sterile drug-mixing syringe and the menstruum separately positioned type sterile drug-mixing syringe overcome the shortcomings of the existing syringe for medical use, having at least the following advantages over prior art:
1. The menstruum, the solute and the syringe of the menstruum prepositioned type sterile drug-mixing syringe form a whole part thus simplifying the drug-mixing and the drug-adding procedure to shorten the preparation time, which is crucial for emergency cases.
2. Similarly, the solute and the syringe of the menstruum separately positioned type sterile drug-mixing syringe also form a whole part thus simplifying the drug-mixing and the drug-adding procedure to shorten the preparation time.
3. The menstruum prepositioned type sterile drug-mixing syringe and the menstruum separately positioned type sterile drug-mixing syringe are easy to carry and use, suitable for use and self-rescuing not only in hospitals, but also outside hospitals such as in the battlefield or in moving conditions, such as in moving vehicles, aeroplanes, vessels, etc.
4. The needle loading means is simplified and manufacturing cost is lowered by omitting the communicating needle.
5. The two stage push-pull structure reduces the overall length of the syringe and the packing size, at the same time the maximum travel of the piston is satisfied to ensure all drug solution can be pushed out.
6. The separately positioned menstruum bottle and the simplified one-needle support structure further shorten the overall length of the syringe, making it more easy to pack and carry.
7. The structure, applicability ad the cost effectiveness of the present invention meets the requirements of the development of the industry and the disclosed structure is new.
8. The structure of the syringe of the present invention is technically more advantageous to the existing syringes. The unique structure and function of the present invention is beyond compare to the existing syringes. Therefore the present invention is inventive.
9. As experienced in researching, designing and manufacturing of medical equipment, the applicant of the invention is very familiar with the defects of the syringes for medical use and the present invention is proposed in view of such defects to achieve the expected objects and functions. Therefore the present invention has industrial applicability.

## Claims

1. A menstruum prepositioned type sterile drug-mixing syringe comprising: a shell (9) of the syringe, a needle (5) positioned in the front end of the shell (9), a piston (10) inside the opening at the rear end of the shell (9) and a push-pull assembly connected to the piston (10), **characterized in that:**
the menstruum prepositioned type sterile drug-mixing syringe further comprising a sliding sleeve (2), a menstruum bottle (3), a solute bottle (8) and a needle loading means;
the sliding sleeve (2) being a hollow cylinder, the menstruum bottle (3) being fixedly socketed in the front portion inside the sliding sleeve (2);
the shell (9) being a hollow cylinder, the front portion of the shell (9) being slidably socketed in the rear portion of the sliding sleeve (2);
the solute bottle (8) being a cartridge pre-charged with powder drug, the piston (10) being slidably socketed in the opening bottom of the solute bottle (8), the solute bottle (8) being fixedly socketed in the shell (9);
the mouth of the menstruum bottle (3) facing the mouth of the solute bottle (8), the tow bottles being connected through the needle loading means;
the needle (5) being a hollow needle having piercing tips at both ends, piercing into the needle loading means and fixedly coupled to the needle loading means.

2. The menstruum prepositioned type sterile drug-mixing syringe according to claim 1, **characterized in that** the needle loading means comprising a sliding support (4), a middle support (6) and a needle support (7), in which:
the sliding support (4) comprising two sections of hollow cylinders, the diameter of the front section being greater than the diameter of the rear section and a circular step being formed at the joint of the two sections of hollow cylinders having different diameters, the mouth of the menstruum bottle (3) being tightly engaged with the inner wall of the front portion of the sliding support (4) and being restricted to inner side of the circular step, the rear portion of the sliding support (4) being slidably socketed with the middle support (6);
the middle support (6) being a hollow cylinder, the front portion of the middle support (6) slidably socketed with the sliding support (4), the rear end of the middle support (6) coaxially contacting the front end of the needle support (7);
the sliding support (4) and the middle support (6) being provided with a pair of positioning mechanism (41, 61) engaging with each other;
the needle support (7) being a hollow cylinder, the front end of the needle support (7) being closed having a bore formed in the centre, the needle (5) passing through the bore and fixedly connected to the needle support (7), non-return mechanism (71) being formed on the inner wall at the rear portion of the needle support (7), the rear portion of the needle support (7) slidably socketing with the mouth of the solute bottle (8).

3. The menstruum prepositioned type sterile drug-mixing syringe according to claim 2, **characterized in that** said a pair of positioning mechanism (41, 61) being a circular bulge or groove (41) formed on the surface of the sliding support (4) slidably contacting the middle support (6) and a circular groove or bulge (61) formed on the surface of the middle support (6) slidably contacting the sliding support (4).

4. The menstruum prepositioned type sterile drug-mixing syringe according to claim 2, **characterized in that** said a pair of positioning mechanism (41, 61) being a protrusion or an indent (41) formed on the surface of the sliding support (4) slidably contacting the middle support (6) and an indent or a protrusion (61) formed on the surface of the middle support (6) slidably contacting the sliding support (4).

5. The menstruum prepositioned type sterile drug-mixing syringe according to claim 2, **characterized in that** the non-return mechanism (71) being claws or a collar (71) formed on the inner wall in the rear portion of the needle support (7).

6. The menstruum prepositioned type sterile drug-mixing syringe according to claim 1, **characterized in that** the push-pull means comprising a first stage push-pull (14), a second stage push-pull (13), a handle (15) and a fixing sleeve (12), in which;
the first stage push-pull (14) is a hollow cylinder, a circular step (141) bulging outward is formed on the front end of the first stage push-pull (14), the fixing sleeve (12) having a reducing rear end, the front end of the fixing sleeve (12) being put on the circular step (141) and screwed to or tightly coupled to the rear end of the shell (9) in order to coaxially fix the first stage push-pull (14) to the rear end of the shell (9);
the second stage push-pull (13) being slidably socketed in the first stage push-pull (14), claws (131) being provided on the front end of the second stage push-pull (13), the rear end of the second stage push-pull being fixedly connected to the handle (15).

7. The menstruum prepositioned type sterile drug-mixing syringe according to claim 6, **characterized in that** an indenter (11) having an outer diameter slightly smaller than the inner diameter of the solute bottle (8) being tightly coupled to the front end of the first stage push-pull (14). The central protruding end of the indenter 11 is embedded into the rear end of the piston 10 in the solute bottle 8.

8. The menstruum prepositioned type sterile drug-mixing syringe according to claim 7, **characterized in that** a positioning ring (16) having an outer diameter equal to or slightly smaller than the inner diameter of the shell (9) is covered on the indenter (11).

9. The menstruum prepositioned type sterile drug-mixing syringe according to claim 1, **characterized in that** the menstruum (3) being a vial pre-charged with liquid menstruum and compressed air, or a cartridge pre-charged with liquid menstruum without inner pressure.

10. The menstruum prepositioned type sterile drug-mixing syringe according to any of claim 1 through claim 9, **characterized in that** the front end of the sliding sleeve (2) being provided with a end cap (1), a protrusion or a circular bulge (31) being form in the middle portion inside the sliding sleeve (2) to hold the shoulder of the menstruum bottle (3).

11. A menstruum separately positioned type sterile drug-mixing syringe comprising: a shell (9) of the syringe, a needle (5) positioned in the front end of the shell (9), a piston (10) inside the opening at the rear end of the shell (9) and a push-pull assembly connected to the piston (10), **characterized in that:**
the menstruum separately positioned type sterile drug-mixing syringe further comprising a menstruum bottle (3), a solute bottle (8) and a needle loading means;
the shell (9) being a hollow cylinder;
the solute bottle (8) being a cartridge pre-charged with powder drug, the piston (10) being slidably socketed in the opening bottom of the solute bottle (8), the solute bottle (8) being fixedly socketed in the shell (9), the mouth of the solute bottle (8) facing forwardly;
the menstruum bottle (3) being separately positioned with other parts;
the rear end of the needle loading means being slidably socketed on the mouth of the solute bottle (8);
the needle (5) being a hollow needle having piercing tips at both ends, piercing into the needle loading means and fixedly coupled to the needle loading means.

12. The menstruum separately positioned type sterile drug-mixing syringe according to claim 11, **characterized in that** the syringe further comprising a sliding sleeve (2), the inner wall at the rear portion of the sliding sleeve (2) slidably engaging with the outer wall at the front portion of the shell (9), the menstruum bottle (3) being fixedly socketed in the front portion of the sliding sleeve (2), the mouth of the menstruum bottle (3) facing backwardly.

13. The menstruum separately positioned type sterile drug-mixing syringe according to any of claim 12, **characterized in that** the front end of the sliding sleeve (2) being provided with a end cap (1), a protrusion or a circular bulge (31) being form in the middle portion inside the sliding sleeve (2) to hold the shoulder of the menstruum bottle (3).

14. The menstruum separately positioned type sterile drug-mixing syringe according to any of claim 12, **characterized in that** it further comprising a packing body (18), the sliding sleeve (2) and the menstruum bottle (3) fixed in the sliding sleeve (2) being put in a recess (19) of the packing body (18), and other components of the syringe being put in a recess (20) of the packing body (18).

15. The menstruum separately positioned type sterile drug-mixing syringe according to claim 11, **characterized in that** the needle loading means comprising a needle support (7), in which the needle support (7) being a hollow cylinder, the front end of the needle support (7) being closed having a bore formed in the centre, the needle (5) passing through the bore and fixedly connected to the needle support (7), non-return mechanism (71) being formed on the inner wall at the rear portion of the needle support (7), the rear portion of the needle support (7) slidably socketing with the mouth of the solute bottle (8).

16. The menstruum separately positioned type sterile drug-mixing syringe according to claim 15, **characterized in that** the non-return mechanism (71) being claws or a collar (71) formed on the inner wall in the rear portion of the needle support (7).

17. The menstruum separately positioned type sterile drug-mixing syringe according to claim 11, **characterized in that** the push-pull means comprising a first stage push-pull (14), a second stage push-pull (13), a handle (15) and a fixing sleeve (12), in which;
the first stage push-pull (14) is a hollow cylinder, a circular step (141) bulging outward is formed on the front end of the first stage push-pull (14), the fixing sleeve (12) having a reducing rear end, the front end of the fixing sleeve (12) being put on the circular step (141) and screwed to or tightly coupled to the rear end of the shell (9) in order to coaxially fix the first stage push-pull (14) to the rear end of the shell (9);
the second stage push-pull (13) being slidably socketed in the first stage push-pull (14), claws (131) being provided on the front end of the second stage push-pull (13), the rear end of the second stage push-pull being fixedly connected to the handle (15).

18. The menstruum separately positioned type sterile drug-mixing syringe according to claim 17, **characterized in that** an indenter (11) having an outer diameter slightly smaller than the inner diameter of the solute bottle (8) being tightly coupled to the front end of the first stage push-pull (14). The central protruding end of the indenter 11 is embedded into the rear end of the piston 10 in the solute bottle 8.

19. The menstruum separately positioned type sterile drug-mixing syringe according to claim 18, **characterized in that** a positioning ring (16) having an outer diameter equal to or slightly smaller than the inner diameter of the shell (9) is covered on the indenter (11).

20. The menstruum separately positioned type sterile drug-mixing syringe according to claim 11, **characterized in that** the menstruum (3) being a vial pre-charged with liquid menstruum and compressed air, or a cartridge pre-charged with liquid menstruum without inner pressure.

21. The menstruum separately positioned type sterile drug-mixing syringe according to any of claim 11 through claim 20, **characterized in that** the needle (5) is covered with a protection jacket (17).
